# EUROPEAN PATENT SPECIFICATION

(11) **EP 3 285 071 B1**
(45) Date of publication and mention of the grant of the patent: **28.07.2021**
(21) Application number: 16780296.6
(22) Date of filing: 14.04.2016
(51) Int. Cl.: G01N 33/68, G01N 33/569

(54) **USE OF BIOMARKER COMPOSITION OR KIT FOR DIAGNOSING STILL'S DISEASE AND DIAGNOSTIC METHOD**
VERWENDUNG VON BIOMARKERZUSAMMENSETZUNGEN ODER KIT ZUR DIAGNOSE VON MORBUS STILL UND DIAGNOSEVERFAHREN
UTILISATION D'UNE COMPOSITION DE BIOMARQUEUR OU TROUSSE DE DIAGNOSTIC POUR LE DIAGNOSTIC DE LA MALADIE DE STILL ET PROCÉDÉ DE DIAGNOSTIC

(30) Priority: 15.04.2015 KR 20150053231; 11.04.2016 KR 20160044090
(43) Date of publication of application: 21.02.2018
(73) Proprietor: Ajou University Industry-Academic Cooperation Foundation and Technology, Suwon-si, Gyeonggi-do 16499 (KR)
(72) Inventor: SOHN, Seonghyang, Suwon-si Gyeonggi-do 16699 (KR); KIM, Hyoun Ah, Suwon-si Gyeonggi-do 16534 (KR)
(74) Representative: Roos, Peter
(86) International application number: PCT/KR2016/003898
(87) International publication number: WO 2016/167573

(56) References cited:
- EP-A1- 1 111 049
- WO-A1-00/52471
- WO-A1-2011/138506
- KR-A- 20140 093 901
- DER-YUAN CHEN ET AL: "Germinal center kinase-like kinase (GLK/MAP4K3) expression is increased in adult-onset Still's disease and may act as an activity marker", BMC MEDICINE, BIOMED CENTRAL LTD., LONDON, GB, vol. 10, no. 1, 6 August 2012 (2012-08-06) , page 84, XP021133203, ISSN: 1741-7015, DOI: 10.1186/1741-7015-10-84
- PARK JEONG HA ET AL: "Natural Killer Cell Cytolytic Function in Korean Patients with Adult-onset Still's Disease", JOURNAL OF RHEUMATOLOGY, vol. 39, no. 10, October 2012 (2012-10), pages 2000-2007, XP9507396,
- Becton Dickinson: "CD11b(D12)", , February 2014 (2014-02), XP055498555, Retrieved from the Internet: URL:http://www.bdbiosciences.com/ds/europe /tds/23-7849.pdf [retrieved on 2018-08-08]
- MINOO ADIB ET AL: "Evaluation of CD11b expression on peripheral blood neutrophils for early detection of neonatal sepsis.", IRAN J ALLERGY ASTHMA IMMUNOL, vol. 6, no. 2, 1 June 2007 (2007-06-01), pages 93-96, XP055498838,
- Thermo Scientific: "Human FcGR2BC (CD32 B/C) ELISA Kit", , 10 February 2015 (2015-02-10), pages 1-8, XP055522346, Retrieved from the Internet: URL:https://assets.thermofisher.com/TFS-As sets/LSG/manuals/EHFCGR2B.pdf [retrieved on 2018-11-08]
- JIN-HYUN WOO ET AL: "Association of Fc[gamma] Receptor Polymorphisms with Adult Onset Still's Disease in Korea", JOURNAL OF RHEUMATOLOGY, vol. 36, 1 January 2008 (2008-01-01), pages 347-350, XP055520995, CA ISSN: 0315-162X, DOI: 10.3899/jrheum.071254
- KOMIYA A ET AL: "Neutrophil CD64 is upregulated in patients with active adult-onset Still's disease", SCANDINAVIAN JOURNAL OF RHEUMATOLOGY, ALMQVIST & WIKSELL PERIODICAL CO., STOCKHOLM, SE , vol. 41, no. 2 1 January 2012 (2012-01-01), pages 156-158, XP009507344, ISSN: 0300-9742, DOI: 10.3109/03009742.2011.644325 Retrieved from the Internet: URL:http://www.informahealthcare.com/journ al/rhe
- SUNG-JI LEE ET AL: "Natural killer T cell deficiency in active adult-onset Still's disease: Correlation of deficiency of natural killer T cells with dysfunction of natural killer cells", ARTHRITIS & RHEUMATISM, vol. 64, no. 9, 1 September 2012 (2012-09-01), pages 2868-2877, XP055521237, US ISSN: 0004-3591, DOI: 10.1002/art.34514
- P EFTHIMIOU: "Diagnosis and management of adult onset Still's disease", ANNALS OF THE RHEUMATIC DISEASES, vol. 65, no. 5, 1 May 2006 (2006-05-01), pages 564-572, XP055271690, GB ISSN: 0003-4967, DOI: 10.1136/ard.2005.042143
- JUNG, YOON SUK ET AL.: 'A Case of Adult-onset Still's Disease Combined with Sjogren's Syndrome' THE KOREAN JOURNAL OF INTERNAL MEDICINE vol. 77, no. 1, 2009, pages S240 - S244, XP009507331
- YAMAGUCHI, M. ET AL.: 'Preliminary Criteria for Classification of Adult Still's Disease' THE JOURNAL OF RHEUMATOLOGY vol. 19, no. 3, March 1992, pages 424 - 430, XP009507351
- SEUNG, O. P. ET AL.: 'Adult-onset Still's Disease: a Case Report' OMAN MEDICAL JOURNAL vol. 26, no. 5, 01 September 2011, pages 1 - 3, XP055497994 DOI: 10.5001/OMJ.2011.96

## Description

### TECHNICAL FIELD

The present invention relates to the use of a biomarker composition for diagnosing Still's disease, the use of a diagnostic kit, and a method of providing information for diagnosis.

### BACKGROUND ART

Still's disease which is the systemic form of juvenile rheumatoid arthritis is a disease that has not yet elucidated its cause since the disease was first described by Dr. Still, and the disease occurs in both children and adults. Adult-onset Still's disease occurring in adults was first reported by Bywaters in 1971, and since then, it has been reported in all around the world and is known as a rare disease or a causative disease of fever of unknown origin.

Adult-onset Still's disease has been usually reported as a benign disease having a favorable prognosis. However, fatal cases where some patients die from adult-onset Still's disease have also been reported. Patients may be in a condition where the patients may die in the case of hemophagocytotic syndrome, worsening liver function, and liver infection. Domestic studies have also reported a prognosis of death in about 10% of the affected patients.

A diagnosis of Still's disease is mainly based on clinical findings. The disease is diagnosed through clinical findings, such as high fever, arthralgia or arthritis, characteristic skin lesion, lymph node enlargement, hepatomegaly, splenomegaly, sclerosis and sore throat, and laboratory findings, such as leukocytosis, accelerated erythrocyte sedimentation, a negative antinuclear antibody, and a negative rheumatoid factor.

Most of the studies on adult-onset Still's disease are associated with inflammatory cytokines using serum of a patient, a simple enzyme-linked immunospecific assay (ELISA) study with respect to an acute reactant material, or a genetic research, but there has been no report of systematic biomarkers. Furthermore, due to lack of understanding of the pathogenesis, the treatment of the disease depends on experience, and there is no consensus thereon.

According to a genetic research of adult-onset Still's disease, it has been reported that the disease is associated with a human leucocyte antigen (HLA), specifically with HLA-DR4 and HLA-DR7, HLA-Bw35, HLA-Cw4, and the like. However, there has been a case of adult Still's disease occurring in only one of an enzygotic twin reported by foreign researchers, and thus, environmental factors are being more focused than genetic factors.

In addition, it has been also suggested that the disease is associated with a virus, and in this regard, human parvo virus B19, cytomegalovirus, rubella virus, echovirus 7, or the like has been reported. It has been also suggested that the disease is associated with *Toxoplasma gondii* and *Yersinia enterocolitica.*

The association with the bacteria above or viral infections is also a reflection of the importance of innate immunity in the pathogenesis of adult-onset Still's disease. The studies of the pathogenesis of adult-onset Still's disease are very limited to cytokine changes in inflammatory state of acute phase and to inflammatory cells, and Interleukin-1 (IL-1), tumor necrosis factor-a (TNF-α), IL-6, and IL-18 are known as related cytokines.

Viral and bacterial infections are considered as causative factors of the pathogenesis of adult-onset Still's disease. However, there is still little research on how these infections cause adult-onset Still's disease.

A pattern recognition receptor that plays an important role in recognizing a damage-associated molecular pattern (DAMP) and a microbe-associated molecular pattern (MAMP) in the early stages of the innate immunity currently attracts attention as an important key. However, there has been no report of changes in such a pattern recognition receptor in a patient with adult-onset Still's disease.

WO 00/52471 A1 is concerned with a rapid assay for chorioamnionitis (infection of the amniotic fluid, fetal membranes, and uterus during labor) in pregnant women that is used for deciding whether to treat the woman and/or her newborn with antibiotics. Becton Dickinson: "CD11b(D12)" is concerned with a monoclonal mouse anti-human reagent for identification of cells expressing CD11b antigen. WO 2011/138506 A1 is concerned with a method for distinguishing a bacterial infection from a viral infection. Thermo Scientific: "Human FcGR2BC (CD32 B/C) ELISA Kit", pages 1-8 concerns instructions about the Human FcGR2BC (CD32 B/C) ELISA Kit.

### DETAILED DESCRIPTION OF THE INVENTION TECHNICAL PROBLEM

The present invention has thus an object in providing the use of a biomarker composition that can diagnose Still's disease.

The present invention also has an object in providing the use of a kit that can diagnose Still's disease.

The present invention also has an object in providing a method of providing information for diagnosis of Still's disease.

### TECHNICAL SOLUTION

To achieve the objects above, the present invention provides the use of a biomarker composition for diagnosing Still's disease, the biomarker composition including at least the protein CD32, as defined in claim 1.

To achieve the objects above, the present invention also provides the use of a kit for diagnosing Still's disease, the kit including an antibody specifically binding to a CD32 cell-surface antigen, as defined in claim 2.

To achieve the objects above, the present invention provides a method of providing information for diagnosis of Still's disease as defined in claim 4, the method including: measuring an expression level of at least the protein CD32 in a sample; comparing the measured expression level with a protein expression level of a normal control sample; and diagnosing Still's disease, when the expression level of CD32 in the sample is higher than that of the protein expression of the normal control sample.

Also disclosed is a method of providing information for diagnosis of Still's disease, the method including: measuring a frequency ratio of granulocytes to lymphocytes in a sample; comparing the measured frequency ratio with a frequency ratio of a normal control sample; and diagnosing Still's disease, when the frequency ratio of the granulocytes to the lymphocytes in the sample is higher than that of the granulocytes to the lymphocytes in the normal control sample.

### ADVANTAGEOUS EFFECTS OF THE INVENTION

According to the present invention, when peripheral blood mononuclear cells (PBMCs) of a patient with Still's disease are compared with those of a normal person, the PBMCs of the patient show high frequency of cells expressing CD11b and cells expressing CD32, and high frequency ratio of granulocytes to lymphocytes. Such information can be used for providing a biomarker composition for diagnosing Still's disease, the biomarker composition including at least one protein selected from CD11b and CD32, a kit for diagnosing Still's disease, the kit including an antibody specifically binding to a CD11b or CD32 cell-surface antigen, and a method of providing information for diagnosis of Still's disease. Accordingly, the diagnosis of Still's disease can be accurately made in children and adults, thereby further improving the therapeutic effects.

### DESCRIPTION OF THE DRAWINGS

FIG. 1 is a table showing frequencies of living cells (P1%) in peripheral blood mononuclear cells (PBMCs), frequencies of cells expressing CD11b, and frequencies of cells expressing CD32.
FIG. 2 is an image showing frequencies of living cells (P1%) in PBMCs, frequencies of cells expressing CD11b, and frequencies of cells expressing CD32.
FIG. 3 is a table showing ratios of granulocytes, ratios of lymphocytes, and ratios of granulocytes to lymphocytes, in PBMCs.
FIG. 4 is an image showing ratios of granulocytes and ratios of lymphocytes, in PBMCs.

### BEST MODE

While the inventors of the present invention were studying a biomarker capable of diagnosing Still's disease, differences in the frequency of cells expressing CD11b, the frequency of cells expressing CD32 and the frequency ratio of granulocytes to lymphocytes between a normal person and a patient with Still's disease were found.

The frequency refers to the number of cells expressing CD11 b, the number of cells expressing CD32, and the number of frequency occupied by with granulocytes or lymphocytes, with respect to the total number of cells

Therefore, the present invention provides the use of a biomarker composition for diagnosing Still's disease, the biomarker composition including at least the protein CD32.

In an embodiment of the present invention, the frequency of cells expressing CD32 in peripheral blood mononuclear cells (PBMCs) of the patient with Still's disease are each found to be higher than that of cells expressing CD32 in PBMCs of a normal person or a patient with rheumatoid arthritis.

In addition, in one or more embodiments of the present invention, the ratio of granulocytes in PBMCs of the patient with Still's disease was found to be higher than that of granulocytes in PBMCs of a normal person or a patient with rheumatoid arthritis, and the ratio of lymphocytes was found to be lower.

The present invention also provides the use of a kit for diagnosing Still's disease, the kit including an antibody specifically binding to a CD32 cell-surface antigen.

The antibody may be a polyclonal antibody or a monoclonal antibody.

The polyclonal antibody may be prepared by injecting an immunogen, such as CD32 protein or a fragment thereof, into a foreign host according to methods known in the art. The foreign host may include a mammal, such as a mouse, a rat, a sheep, or a rabbit. The immunogen may be injected via an intramuscular, intraperitoneal, or subcutaneous injection method, and is generally administered with an adjuvant to increase antigenicity. Serum is collected periodically from a foreign host to collect serum having improved titer and showing specificity to antigens, or to separate and purify antibodies therefrom.

The monoclonal antibody may be prepared by an immortalized cell line generation technique using a fusion method known in the art. For example, a protein expressed in a gene may be immunized in a mouse, or a peptide may be synthesized and bound to a bovine serum albumin to be immunized in a mouse. Antigen-secreting B lymphocytes separated from the mouse may be then fused with myeloma of a human or a mouse to produce immortalized hybridomas, and the hybridomas may be subjected to an indirect enzyme-linked immunoabsorbent assay (ELISA) to confirm whether the monoclonal antibody is produced. Then, positive clones may be selected and cultured, and antibodies may be separated and purified. Alternatively, antibodies may be injected into the abdominal cavity of a rat, and ascites may be collected, thereby preparing the monoclonal antibody.

The monoclonal antibody may be quantitatively analyzed by performing a color reaction using a secondary antibody to which an enzyme such as alkaline phosphatase (AP) or horseradish peroxidase (HRP) is bound, and a substrate of the secondary antibody. Alternatively, the monoclonal antibody may be directly quantitatively by using an antibody to which an enzyme such as AP or HRP is bound for the monoclonal antibody against the protein above. In addition, an antibody labeled with various fluorescent markers such as fluorescein isothiocyanat (FITC) and phycoerythrin (PE) may be used.

The reaction between the protein above and the antibody may be confirmed by protein identification experiments such as western blot, Immunoprecipitation (IP), ELISA, immunohistochemistry (IHC), and flow cytometry analysis (FACS), but embodiments of the present invention are not limited thereto.

The kit including the antibody may typically include an antibody and a buffer that are in the lyophilized form, a stabilizer, an inactive protein, or the like, wherein the antibody may be labeled with a radionuclide, a fluorescer, an enzyme, or the like.

The present invention also provides a method of providing information for diagnosis of Still's disease, the method including: measuring an expression level of at least the protein CD32 in a sample; comparing the measured expression level with a protein expression level of a normal control sample; and diagnosing Still's disease, when the expression level of CD32 in the sample is higher than that of the protein expression of the normal control sample.

Also disclosed is a method of providing information for diagnosis of Still's disease, the method including: measuring a frequency ratio of granulocytes to lymphocytes in a sample; comparing the measured frequency ratio with a frequency ratio of a normal control sample; and diagnosing Still's disease, when the frequency ratio of the granulocytes to the lymphocytes in the sample is higher than that of the granulocytes to the lymphocytes in the normal control sample.

When the frequency ratio of the granulocytes to the lymphocytes is in a range of about 3.0 to about 50.0, it is diagnosed as Still's disease.

An example of the sample may be blood, and more preferably, may be PBMCs separate from blood, but embodiments of the present invention are not limited thereto.

### MODE OF THE INVENTION

Hereinafter, to promote understanding of one or more exemplary embodiments, reference has been made to the exemplary embodiments. However, the present invention should not be construed as being limited to the exemplary embodiments set forth herein. Rather, these embodiments are provided so that this disclosure will be thorough and complete, and will fully convey the concept of the inventive concept to those skilled in the art.

### <Example 1> Preparation of peripheral blood mononuclear cells (PBMC)

Human PBMCs were separated from human blood of a healthy donor by using an ammonium-chloride-potassium (ACK) lysis buffer. Then, 2 mL of blood was added to a tube treated with ethylenediaminetetraacetic acid (EDTA), and the tube was inverted 15 times. Afterwards, the resultant blood was added to 40 mL of the ACK lysis buffer, and was left at room temperature for 5 minutes. The centrifugation was performed thereon at a speed of 300 xg for 5 minutes. After white blood cells (WBC) were collected, the supernatant was discarded and the precipitated cells were mixed in 5 ml of PBS. The centrifugation was repeatedly performed thereon twice at a speed of 300 xg at a temperature of 4°C, thereby preparing PBMCs.

### <Example 2> Measurement of cells expressing CD11b or cells expressing CD32 in PBMCs

The frequencies of cells expressing CD11b and cells expressing CD32 were each measured in the PBMCs prepared according to Example 1, with respect to normal people (19 people), patients with rheumatoid arthritis (RA) (19 people), and patients with Still's disease (18 people).

Here, red blood cells were removed from each blood, and leukocytes were collected. Then, by a reaction with anti-CD11b and anti-CD32 antibodies each labeled with different fluorescence, the leukocytes were reacted with the antibodies, and the number of cells to which the antibodies bound was counted according to FACS. In this regard, the frequency of these cells occupied over the entire cells was analyzed.

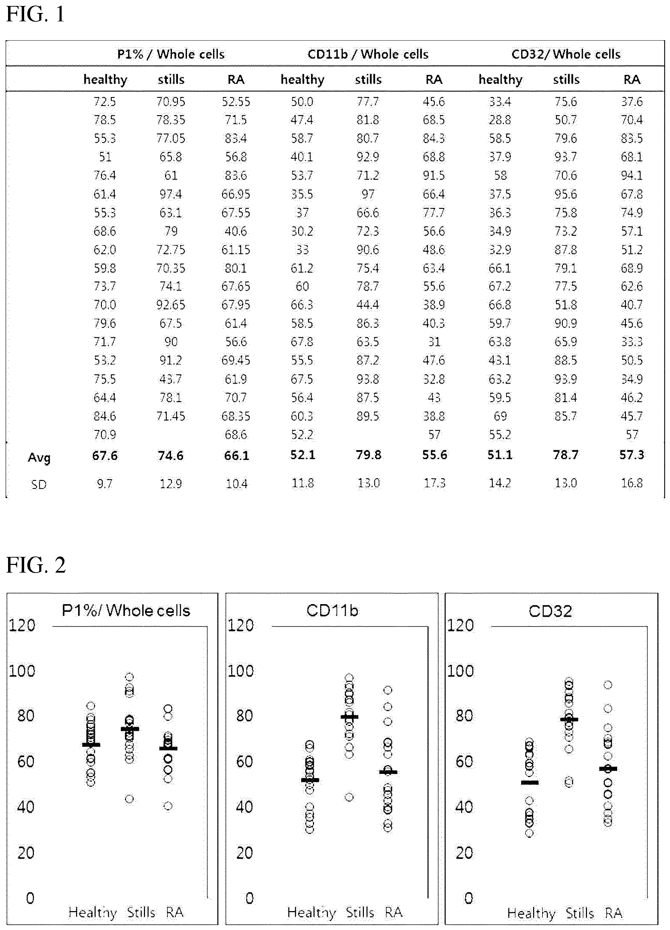

Consequently, as shown in FIGS. 1 and 2, it was confirmed that the frequencies of the cells expressing CD11 b and the cells expressing CD32 were significantly high when compared with the normal control group and the test group of patients with RA.

Here, there was no bid difference overall regarding the frequency of living cells over the entire cells (P1%).

### <Example 3> Measurement of frequencies of granulocytes and lymphocytes in PBMCs

In the same manner as in Example 2, the frequencies of granulocyte and lymphocyte and the ratio of granulocytes to lymphocytes were measured with respect to normal people (19 people), patients with RA (19 people), and patients with Still's disease (18 people).

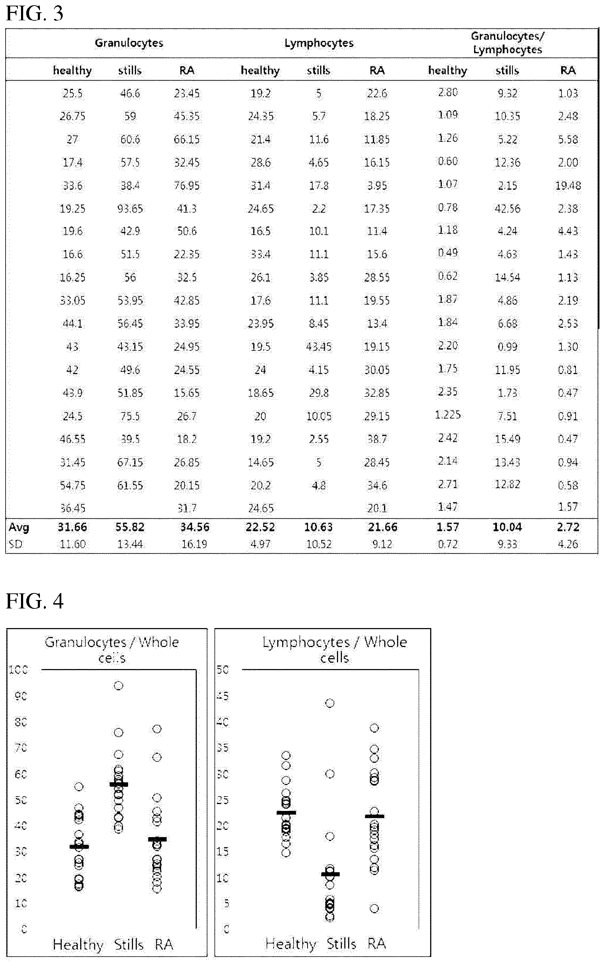

Consequently, as shown in FIGS. 3 and 4, it was confirmed that the frequency of granulocytes of the group of patients with Still's disease was higher than that of granulocytes of the normal control group and the test group of patients with RA, and that the frequency of lymphocyte of the group of patients with Still's disease was significantly lower than that of lymphocyte of the normal control group and the test group of patients with RA.

In particular, referring to FIG. 3, as a result of confirming the ratio of granulocytes to lymphocytes, the ratio of the group of patients with Still's disease was about 7 times higher than that of the normal control group, and accordingly, it was confirmed that the ratio of granulocytes was significantly high in PBMCs of the patients with Still's disease.

Overall, when the PBMCs of the patients with Still's disease are compared with those of the normal control group, the frequencies of the cells expressing CD11b and the cells expressing CD32 were high, as well as the ratio of granulocytes to lymphocytes. Thus, through analysis, the present invention can be utilized for the diagnosis of Still's disease.

## Claims

1. Use of a biomarker composition for in vitro diagnosis of Still's disease, the biomarker composition comprising at least the protein CD32.

2. Use of a kit for in vitro diagnosis of Still's disease, the kit comprising an antibody specifically binding to a CD32 cell-surface antigen.

3. Use of a kit of claim 2, wherein the antibody is a monoclonal antibody or a polyclonal antibody.

4. A method of providing information for diagnosis of Still's disease, the method comprising:
measuring an expression level of at least the protein CD32 in a sample, wherein the sample is a peripheral blood mononuclear cell (PBMC);
comparing the measured expression level with a protein expression level of a normal control sample; and
diagnosing Still's disease, when the expression level of CD32 in the sample is higher than that of the protein expression of the normal control sample.

5. The method of claim 4, wherein the sample is blood.

## Patentansprüche

1. Verwendung einer Biomarkerzusammensetzung zur In-vitro-Diagnose von Morbus Still, wobei die Biomarkerzusammensetzung mindestens das Protein CD32 umfasst.

2. Verwendung eines Kits zur In-vitro-Diagnose von Morbus Still, wobei das Kit einen Antikörper umfasst, der spezifisch an ein CD32-Zelloberflächenantigen bindet.

3. Verwendung eines Kits nach Anspruch 2, wobei der Antikörper ein monoklonaler Antikörper oder ein polyklonaler Antikörper ist.

4. Verfahren zur Bereitstellung von Informationen für die Diagnose von Morbus Still, wobei das Verfahren umfasst:
Messen eines Expressionsniveaus von mindestens dem Protein CD32 in einer Probe, wobei die Probe eine periphere mononukleäre Blutzelle (PBMC) ist;
Vergleichen des gemessenen Expressionsniveaus mit einem Proteinexpressionsniveau einer normalen Kontrollprobe; und
Diagnostizieren des Morbus Still, wenn das Expressionsniveau von CD32 in der Probe höher ist als das der Proteinexpression der normalen Kontrollprobe.

5. Verfahren nach Anspruch 4, wobei die Probe Blut ist.

## Revendications

1. Utilisation d'une composition de biomarqueur pour le diagnostic in vitro de la maladie de Still, la composition de biomarqueur comprenant au moins la protéine CD32.

2. Utilisation d'un trousse de diagnostic pour le diagnostic in vitro de la maladie de Still, la trousse comprenant un anticorps se liant spécifiquement à un antigène de surface cellulaire CD32.

3. Utilisation d'un trousse selon la revendication 2, dans laquelle l'anticorps est un anticorps monoclonal ou un anticorps polyclonal.

4. Procédé pour fournir des informations pour le diagnostic de la maladie de Still, le procédé comprenant :
mesurer un niveau d'expression d'au moins la protéine CD32 dans un échantillon, dans lequel l'échantillon est une cellule mononucléaire du sang périphérique (PBMC) ;
comparer le niveau d'expression mesuré avec un niveau d'expression de protéine d'un échantillon de contrôle normal ; et
diagnostiquer la maladie de Still, lorsque le niveau d'expression de CD32 dans l'échantillon est supérieur à celui de l'expression de la protéine de l'échantillon de contrôle normal.

5. Procédé selon la revendication 4, dans lequel l'échantillon est du sang.
